# EUROPEAN PATENT APPLICATION

(11) **EP 3 385 730 A1**
(43) Date of publication of application: **10.10.2018**
(21) Application number: 18165547.3
(22) Date of filing: 03.04.2018
(51) Int. Cl.: G01R 27/02

(54) **HIGH IMPEDANCE SENSING INTEGRATED CIRCUIT**

(30) Priority: 31.03.2017 WO PCT/US2017/479413
(71) Applicant: Senxellion GmbH, 6039 Root (CH)
(72) Inventor: BARRETTINO, DIEGO RUBEN, 6900 LUGANO (CH); ALLEGRI, DANIELE GUIDO, 6702 CLARO (CH); DONIDA, ACHILLE, 6900 LUGANO (CH); MASCIADRI, MARCO, 6900 PARADISO (CH)
(74) Representative: Bettello, Pietro

(57) **Abstract**

The invention concerns a process for measuring the electrical impedance of a system, which includes the following phases:
applying an excitation current to the system with only 3 current levels at different frequencies;
detecting with a low-noise amplifier (LNA) the voltage drop from the system (input signal) caused by the excitation current;
applying a frequency shift to the amplified input signal moving it to an intermediate frequency (f_{IF}), for example, by selecting f_{IF} > flicker noise corner frequency thus limiting the effects of noise;
low-pass filtering (LPF), amplifying (PGA), and then converting to the digital domain (ADC) the frequency shifted signal;
digitally demodulating and filtering the converted signal to obtain the I/Q DC values that represent the electrical impedance of the system.

## Description

of the invention entitled "Process for measuring the electrical impedance of a system" in the name of Senxellion GmbH.

The present invention relates to a process for measuring the electrical impedance of a system, according to the general part of claim 1. The present invention relates also to an impedance measuring chip.

This application claims priority from provisional application serial no. 62/479,413 filed on March 31, 2017, the disclosure of which is hereby incorporated herein by reference in itis entirety.

Impedance analyzers are broadly used to measure the electrical impedance of devices under test and to characterize the physical properties of different materials [1]. The most popular method used to measure the electrical impedance in commercial impedance analyzers is the auto-balancing bridge method. There are also some commercial impedance analyzers that measure the voltage and the current signals directly without needing the feedback loop used by the auto-balancing bridge method. However, these commercial instruments are expensive and consume a significant amount of power.

In the past some have used a low cost low power impedance analyzers based on the AD5933 chip from Analog Devices can be used, which is the only commercial monolithic impedance analyzer available in the market. However, these impedance analyzers can only operate up to 100 kHz due to the limited frequency range of the AD5933 chip.

Therefore, there is a need for a low-cost low-power multifrequency impedance analyzer based on a monolithic mixed-signal (analog/digital) microchip that performs all the tasks necessary to perform impedance measurements in the frequency range from 10kHz to 10MHz.

Thus, there is a new design, which combines a lock-in approach with the dual step super-heterodyne demodulation scheme. In contrast to the full analog approach, a mixed analog/digital solution is adopted. In particular, this solution performs a first frequency down conversion in the analog domain and shifts the I,Q demodulation in the digital domain. This has the big advantage of removing any sensible dual path from the analog domain with important benefits in terms of complexity, precision and power consumption.

There are many possible applications of the High Precision Impedance Sensing Integrated Circuit (HPISic). For example, some of these applications can include the ability to measure with high accuracy the complex impedance seen between two electrodes. In particular, it can be used to monitor the impedance variation of the living tissue for biomedical applications.

In another application, thanks to the multi device configuration, it will allow to measure the voltage drop induced by a master device on a wide tissue (or other material) area.

In another application it will measure with high resolution any voltage/current signal having a bandwidth of 1kHz.

In another application the HPISic device can read out the following biomedical signals: Bioimpedance; Electrocardiogram; Heart Rate; Electromyography; Galvanic skin response; Measurement and frequency characterization of capacitive sensors (industrial application); Evaluation of water quality (environmental application); Evaluation of soil quality (agricultural application).

Other objects and features of the present invention will become apparent from the following detailed description considered in connection with the accompanying drawings which disclose at least one embodiment of the present invention. It should be understood, however, that the drawings are designed for the purpose of illustration only and not as a definition of the limits of the invention.

In the drawings, wherein similar reference characters denote similar elements throughout the several views:
FIG. 1 is a schematic block diagram of a High Precision Impedance Sensing Integrated Circuit (HPISic);
FIG. 2 is schematic block diagram of a mixed analog/digital approach to a HPISic;
FIG. 3A is a heterodyne lock in amplifier;
FIG. 3B is another implementation of the HPISic;
FIG. 4A is a formula for a sine wave;
FIG. 4B is an operation of the sine wave with demodulation;
FIG. 4C is a formula to ensure the correct operation of the HPISic;
FIG. 4D is a formulaic application of the device;
FIG. 5 is a digital block of multiple phase accumulators;
FIG. 6A is a first example of a generated signal;
FIG. 6B is a second example of a generated signal;
FIG. 7 is a view of a top design of the HPISic with different pins;
FIG. 8 is a view of a photograph of a HPISic device;
FIG. 9 shows a view of a HPISic device used to measure bioimpedence values.

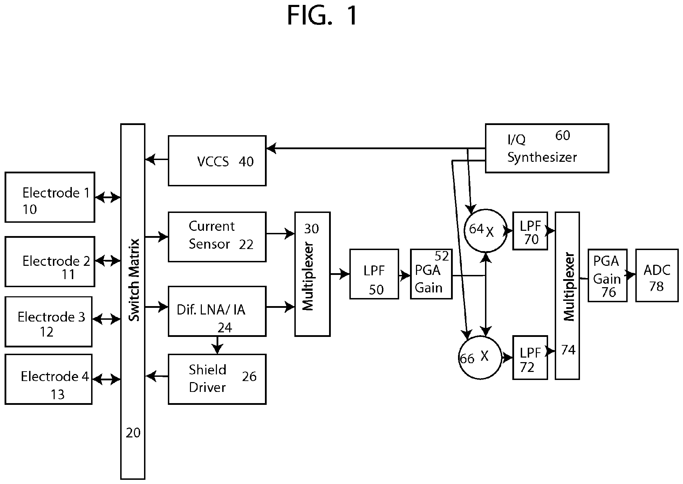

Referring to the drawings, FIG. 1 shows a schematic block diagram of a High Precision Impedance Sensing Integrated Circuit (HPISic).

Thus, there is shown a plurality of electrodes 10, 11, 12, and 13 which each provide an analog input for the circuit. These signals feed into a switch matrix 20.

The switch matrix 20 has outputs that feed into current sensor 22, and low noise amplifier 24. The outputs of low noise amplifier 24 and current sensor 22 feed into multiplexer 30. In addition, the output of low noise amplifier 24 feeds into shield driver 26. The output of shield driver 26 fees back into switch matrix 20. The output of multiplexer 30 fees into lower power filter 50 which feeds into programmable gain amplifier (PGA) 52. The output of programmable gain amplifier 52 feeds into each of two different demodulators 64 and 66, hereinafter also referred to as (Q,I) demodulators.

In this analog implementation, these two demodulators 64 and 66 need to be operated at the same time (due to the extremely long time constant of the low-pass filter 50 in order to correctly acquire the amplitude and the phase of the input signals. As a consequence, the dual path structure of this design may have a negative impact on the on the accuracy of the measured values and on the power requirement.

The output of each of the demodulators 64 and 66 each feed into respective low pass filters 70 and 72, wherein the output of each of these filters feeds into multiplexer 74. The output of multiplexer feeds into a PGA gain 76, wherein the output of PGA gain 76 then feeds into analog to digital converter (ADC) 78.

After the multiplexer 74, all signals are at DC level and any uncompensated offset will reduce the system performance.

The I/Q synthesizer 60 requires two independent high-speed (80MSPS) high-resolution (14-bit) digital to analog converters DACs to synthesize the I and Q sinusoidals. At least one signal from the I/Q synthesizer 60 feeds back to form a voltage-controlled signal 40 that is fed back into the switch matrix 20.

The HPISic circuit includes a lock in amplifier formed by the components indicated above. The operation of a lock-in amplifier relies on the orthogonality of sinusoidal functions. More precisely, when a sinusoidal function of frequency 1 of a first signal is multiplied by another sinusoidal function of frequency 2 not equal to 1 (of a second signal) and integrated over a time much longer than the period of the two functions, the result converges to zero. Instead, when 1 is equal to 2 and the two functions are in phase, the average value is equal to half of the product of the amplitudes.

In essence, a lock-in amplifier takes the input signal, multiplies it by the reference signal and integrates it. The resulting signal is a DC signal, where the contribution from any signal that is not at the same frequency as the reference signal is attenuated close to zero. The out-of-phase component of the signal that has the same frequency as the reference signal is also attenuated.

Although from a conceptual point of view this structure is able to realize the required functionality, it does not represent the optimal solution. Here after are listed the main limiting points.

With this configuration the circuit will be forced to provide high gain (>100dB and linear phase up to 50MHz. This will be traduced in high requirement in terms of circuit complexity and power consumption.

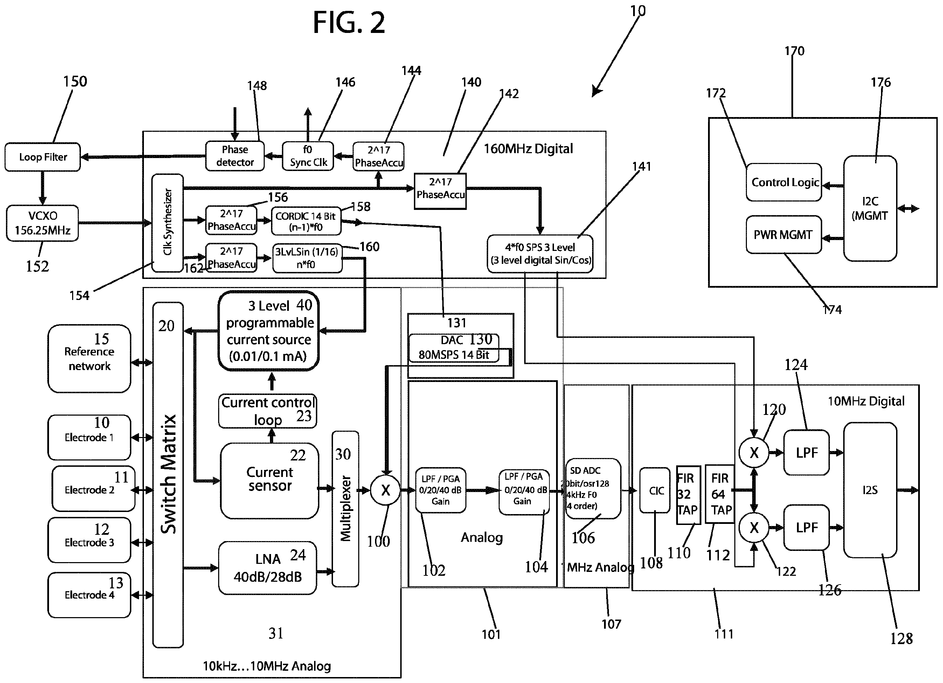

FIG. 2 is schematic block diagram of a mixed analog/digital approach to a HPISic. In contrast to the full analog approach of FIG. 1, a mixed analog/digital solution is adopted in FIG. 2.

The circuit design has both an analog section and a digital section. The analog section comprises subsections, domains or circuits 31, 101, 107, and 131. The digital section comprises subsections, domains, or circuits 111, 140 and 170.

For example, the design includes a plurality of electrodes 10, 11, 12, and 13 as well as well as a reference network which have a bi-directional feed into switch matrix which is housed inside of first analog frequency domain 31. The first analog frequency domain 31 corresponds to the high frequency signal domain (RF) (10kHz ...10MHz). It contains all the front end building blocks of the system. This domain has the highest demand in terms of noise and bandwidth. There we find the low noise amplifier (LNA) 24 and the current stimulation circuit. In addition, also disposed inside of first analog frequency domain 31 are a current sensor 22, a low noise amplifier 24, a current control loop 23, a multiplexer and a multi-level programmable current source 40 which in at least one embodiment is a three level programmable current source.

Switch matrix 20 has outputs that feed into current sensor 22 and low noise amplifier 24. The outputs of current sensor 22 and low noise amplifier 24 feed into multiplexer 30. The output of multiplexer 30 fees into demodulator 100. The output of demodulator 100 feeds into a second analog frequency domain 101.

The second analog frequency domain 101 is the intermediate frequency domain (0.5kHz...1.5 kHz, IF region). It extends from the output of the first demodulator 100 to the input of the ADC (analog to digital controller) 106 (disposed in third analog region 107). It comprises the analog part of the heterodyne lock-in structure. In this domain, the fine gain adjustment and filtering required to fulfill the specified sensitivity are performed. Disposed in this domain 101, are a plurality of low pass filters (LPF)/ programmable gain amplifiers (PGA) 102 and 104 coupled together in series, wherein the input of LPF/PGA 102 is from demodulator 100 while the output of LPF/PGA 104 is into analog to digital converter (ADC) 106.

ADC 106 is disposed inside of the third analog frequency domain 107 (1MHz), which is the domain where the ΣΔ ADC (Sigma Delta modulator) 106 operates.

In the fourth analog region there is also a fourth analog frequency domain 131 which comprises the digital to analog converter (DAC) 130. With its 80MSPS, DAC 130 represents the fastest analog circuit of the system.

The output of DAC 130 feeds into demodulator 100 while the output of a first digital domain 140 feeds into DAC.

Thus, there are two separate regions analog, and digital wherein this design performs a first frequency down conversion in the analog domain and shifts the I, Q demodulation in the digital domain. This has the big advantage of removing any sensible dual path from the analog domain with important benefits in terms of complexity, precision and power consumption. A key point of the structure is the synchronicity between the signals, where all signals are multiple of a reference frequency f0. In particular, we have: current source output frequency (f_{RF}) which is n* f0, DAC demodulator frequency (fr) which is (n-1)*f0, intermediate frequency (f_{IF}) which is f0, ADC sampling frequency (fs) which is 4* f0 and the IQ demodulation reference frequency (fI) which is 4* f0.

Thus, the analog domain comprises all the circuits required to generate all stimulation currents and collect the induced voltage signal and comprises all circuits necessary to perform the first step of the lock-in detection. The digital domain comprises the synchronous signal generator, the ADC decimator, the digital part of the lock-in detector, and all control logic required to manage the chip and to communicate with an external controller. Thus, each domain has different requirements in terms of operating frequency and bandwidth.

The first digital frequency domain 140, which operates at the highest frequency (max operating frequency 160 MHz), is responsible of generating all synchronous clocks and the digital sinusoid required by the dual-step lock-in operation. In particular, in this domain are located all the phase accumulators and the CORDIC digital sinus generator. With this first digital frequency domain 140 there is an external voltage controlled crystal oscillator (VCXO) 152 which receives a signal from loop filter 150. VCXO 152 feeds into synthesizer 154 which therein feeds two separate signals into two different phase accumulators 156 and 162. The output of phase accumulator 156 is into CORDIC 158 while the output of phase accumulator 162 is ultimately into the divider 160 that feeds the three-level programmable current source 40.

The output of CORDIC 158 is into DAC 130 in domain131. In addition, an output of synthesizer 154 is also into phase actuators 142 and 144. The output of phase detector 144 is into sync clock 146 which has an output into phase detector 148. The output of phase detector 148 is into loop filter 150.

In addition, the output of phase actuator 142 is into three level digital sin/cos circuit 141. The two outputs of this circuit 141 is into each of demodulators 120 and 122 in a second digital frequency domain 111.

The second digital frequency domain 111, operates with maximal operating frequency of 10MHz, is responsible for the IQ demodulation and filtering. In particular, in this domain, we find: the sampling frequency converter 108 which receives an input from ADC 106 in domain 107 and translates the 1-bit bit stream into a 4kSPS 20-bit digital signal. The output of the frequency converter 108 feeds into first a 32 tap FIR filter 110. The output of the 32 tap FIR filter fees into the 64 tap FIR filter 112. The output of the 64 tap FIR filter feeds into both demodulators 120 and 122. The output of demodulator 120 feeds into 128 tap FIR filter 124 while the output of demodulator 122 feeds into a second 128 tap FIR filter 126. The output of these tap filters 124 and 126 feed into circuit 128.

The third digital frequency domain 170, which contains all the digital components that work with a maximum frequency of 1MHz, comprises the management and communication domain. It includes all the control logic 172, which manages the chip, and all the communication logic 176, which handles the communication with the external microcontroller. This also comprises the power management of the chip 174. This third digital frequency domain 170 mainly provides the clocks for the blocks used for control logic, power management and communication with the external microcontroller.

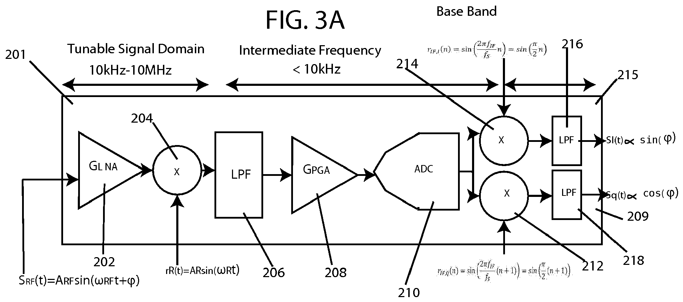

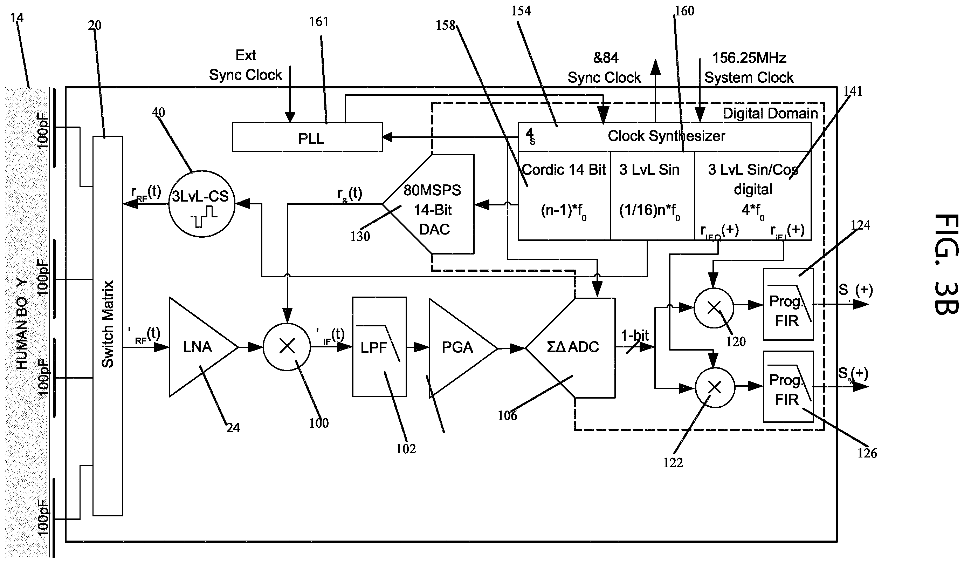

FIG. 3A shows a new and alternative idea to the full analog classical approach, with this design, the input signal is first frequency shifted to the intermediate frequency (f_{IF}) domain, low pass filtered (LPF) and then sampled by an analog-to-digital converter (ADC). At the end, the digital sampled signal is digitally demodulated to DC. With this solution, the phase detector is fully implemented in the digital domain.

Thus, with this design there is a combined circuit 200 which has an analog section 201 and a digital section 209. In the analog section a low noise amplifier 202 receives an input signal, this signal is then passed onto demodulator 204. The output of demodulator 206 is passed into PGA 208. The signal from PGA 208 is then passed into ADC 2310. The output of ADC 210 is split into separate demodulators 212 and 214. The output of separate demodulator 212 passes into low pass filter 218, while the output of demodulator 214 passes into low pass filter 216.

Compared to the full analog approach, the digital phase detector has the great advantage of being completely immune to offset problems and non-linearities.

Regarding to the impact on the complexity of the system, the greatest impact is at the level of the ADC converter such as converter 210, which is forced to operate at the intermediate frequency. This, although it might seem full of the implementation difficulties, actually turns out to be a major simplification. Indeed, when dealing with noise and offsets, most of the analog circuits are forced to implement chopping and active offset compensation techniques. On the other hand, by sampling the signal directly at the intermediate frequency, these techniques reveal to be unnecessary. In fact, in the IF domain, the signal of interest is enclosed in the AC component of the signal. This makes the detector virtually immune to all DC components present or added to the signal. It should also be noted that by placing the intermediate frequency appropriately, it is possible to inherently limit the effects of noise (e.g. by placing f_{IF} flicker noise corner frequency). Concerning the sampling rate, the fact of operating at the intermediate frequency instead of DC does not represent a particular limit. In fact, the frequency fIF of the system will be located in the lower half of the audio band 0Hz < f_{IF} < 10kHz < 24kHz.

The digital demodulation normally requires complex digital circuits, which are capable of performing full precision multiplications. In general, floating-point units are implemented to minimize the effects of rounding and approximation. Instead, for this specific application the demodulation step can be achieved in a very simple way without losing a single bit of precision in the whole process.

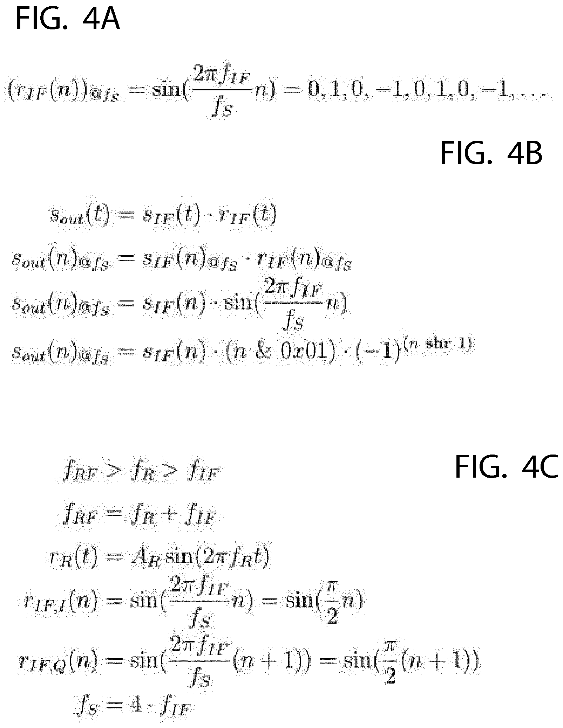

FIG. 4A shows the particular case of an ideal sine wave sampled at 4 times its fundamental frequency (fs = 4 • f_{IF}). FIG. 4B shows the operation of demodulation can be transformed into signal out formula.

From a digital implementation point of view, this operation requires nothing more than a simple inversion of sign combined with some logic gates. From the mathematical point of view, however, this approach has the valuable property to operate an ideal demodulation without adding any inaccuracy due to the digital quantization and due to the digital multiplication. In other words, this means that the accuracy of this phase detector is only determined by the resolution of the ADC.

In addition, apart from the aspect related to the precision in the demodulation, this system turns out to be also optimal from the point of view of shared structures. In particular, thanks to the low sampling frequency, this solution allows to use the technique of time-multiplexing resource sharing and implement a double demodulator with a single digital structure. The first demodulator will decode the in-phase signal (I demodulator), and the second demodulator will decode the quadrature signal (Q demodulator). This always ensures absolute consistency between the I and Q samples, and thus also guaranteeing the highest possible accuracy in the detection of the module and phase of the incoming signal.

FIG. 4C shows the circumstances necessary to ensure the correct operation of the system.

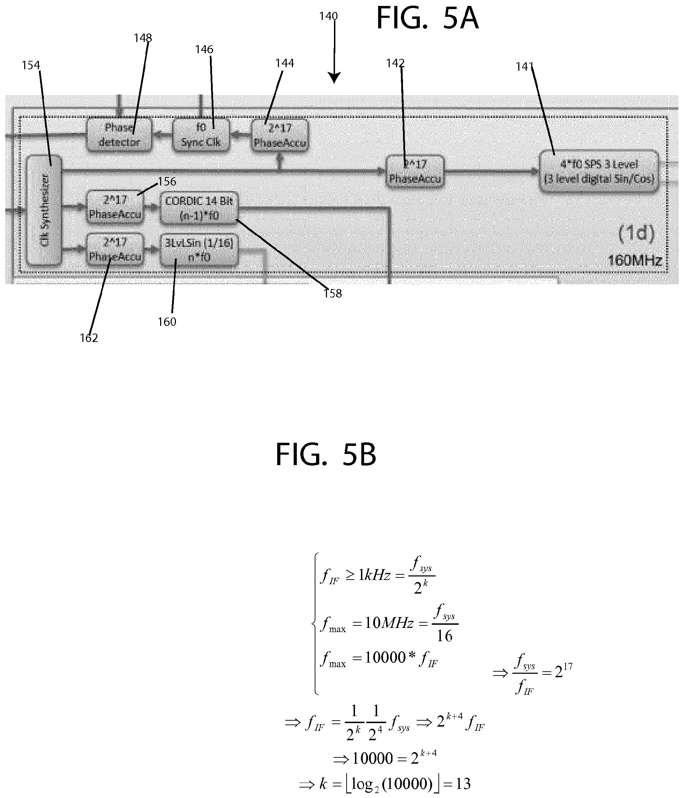

FIG. 5A shows a close up view of the first digital domain 140 which is responsible for generating all clock signals according to the above rules named clock synthesizer 154.

The clock synthesizer is a digital block composed by 5 phase accumulators, which are able to provide 5 different signals at independent programmable frequency and phase.

Furthermore, in order to allow a multi device operation, the clock synthesizer 154 is able to synchronize to an external reference clock by means of a dedicated pin.

In order for the phase accumulators to cover the wider operating frequency range and to ensure the phase coherency between all the generated sinusoidals, the IF frequency was selected to be a 1/2K factor of the system clock. Considering an oversampling factor for the CORDIC sinus generator 158 equal to 8 and an oversampling ration of 16 for the RF signal generator, this formula is shown in FIG. 5B, wherein f_{sys} is limited by two factors: process and maximal frequency, which can be synthesized. Considering these limitations, and also considering the available VCXO 152 on the market, we set f_{sys}=156.25 MHz. Then, the calculated working frequencies are shown in Table 1.

**Table 1: Frequency constrains**

| FREQUENCY | VALUE |
|---|---|
| f_{sys} | 156.25MHz |
| f_{IF} | 1.19kHz |
| f_{RFmax} | 9.77MHz (n=8192) |
| f_{RFmin} | 5.96kHz (n=5 |
| f_{minimal step} | 1.19kHz |

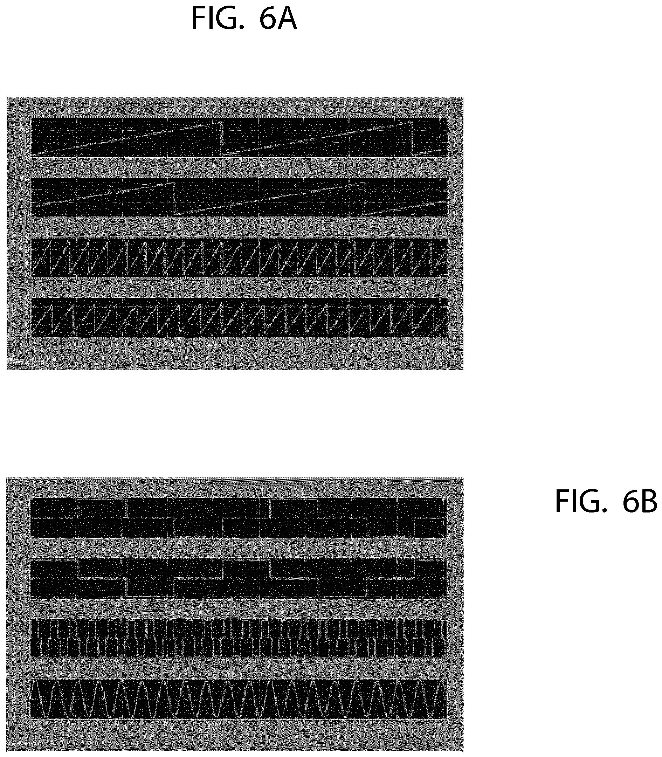

FIGS. 6A and 6B show an example (n=10) of the generated signals. Starting from the top of each figure, we have: (IF domain) I phase accumulator/3 level sinusoidal, (IF domain) Q phase accumulator/3 level sinusoidal, (RF domain) (n*f_{IF}) phase accumulator/ 3 level sinusoidal, (RF domain) ((n 1) *f_{IF}) phase accumulator/ ADC sinusoidal.

Regarding the complexity and the criticality of the system, the classic single demodulation stage lock-in amplifier approach is therefore different from at least one embodiment of the present invention in that the present invention is configured to reduce the number of analog multipliers from two do one. The present invention is configured to avoid inaccuracies due to mismatch between dual path structures (I/Q demodulator). The present invention is configured to create a more robust system, which is intrinsically insensitive to DC offset errors added by the analog elaboration chain. The present invention is configured to limit the gain of the input of the system to the minimum required in order to achieve the noise specifications (High Bandwidth, Low Gain), and shift and processing into the fixed IF frequency domain (Low Bandwidth, Variable High Gain) and all this, with a potential positive impact on power consumption and on the occupied silicon area.

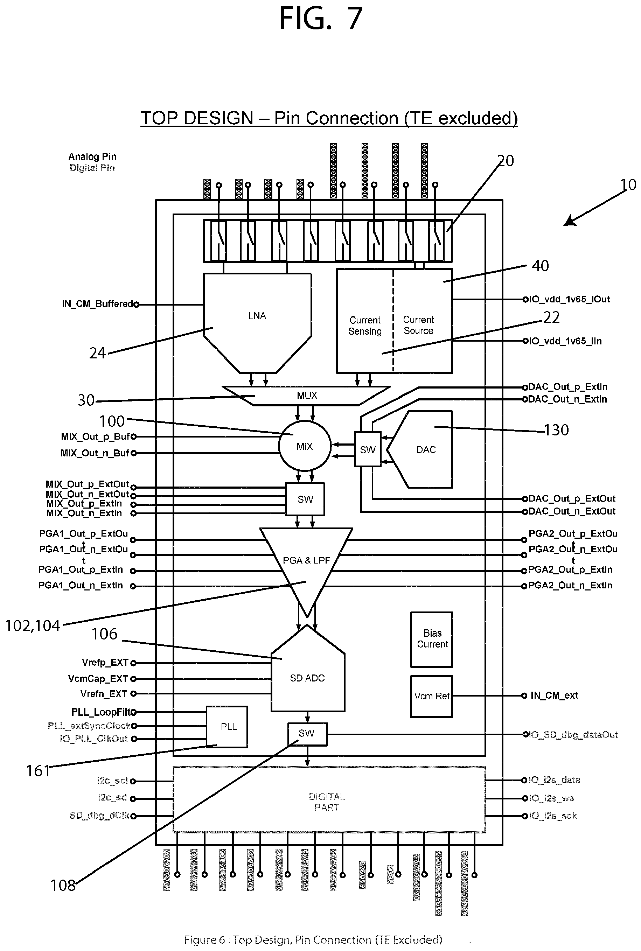

FIG. 7 is the top design block diagram of the HPISic device showing in detail the different pins used for operating, debugging and testing the device. This view shows the analog portion of the circuit or chip in detail. For example, there is shown switch matrix 20 which feeds into low noise amplifier 24. Current sensing circuit and current source 40 feed into muxer or multiplexer 30. The output of multiplexer 30 is into demodulator 100. In addition, DAC 130 feeds into demodulator 100 as well. The output of demodulator feeds into PGA/LPF 102 and 104 (see also FIG. 2). This then feeds into Sigma-Delta ADC 106. The output of Sigma-Delta ADC 106 feeds into sampling converter 108 which feeds into the digital part of the chip. In addition, disposed on this chip is a phase locked loop 161.

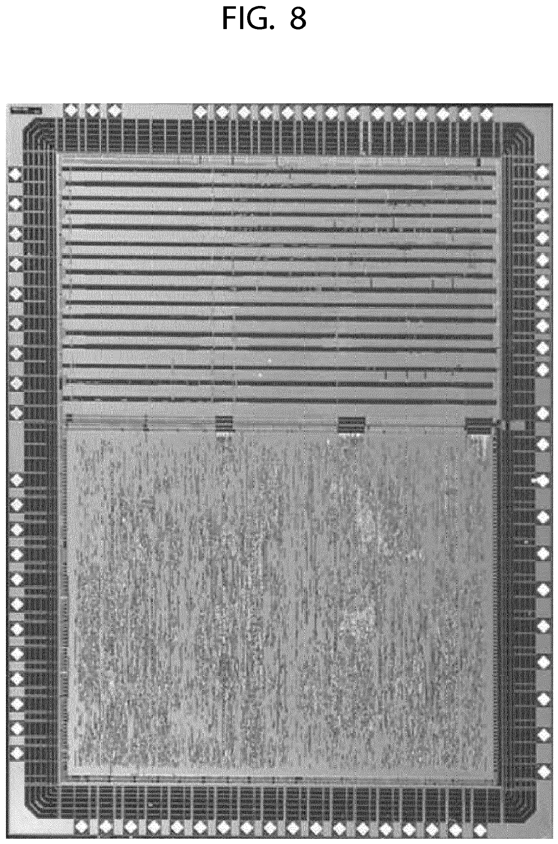

FIG. 8 is a picture of the HPISic device.

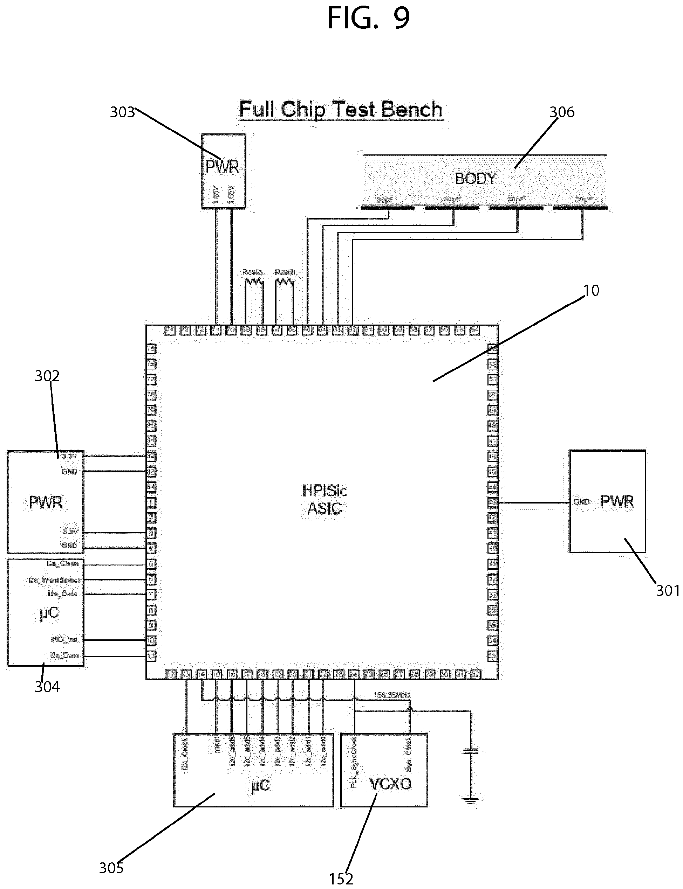

FIG. 9 is a full chip bench test used to measure the tissue impedance thus making it possible the estimation of the hydration level of the subject under test. With this design there is HPISic chip 10 which is coupled to external components such as different power sources 301, 302, and 303. In addition, coupled to chip 10 is a voltage controlled crystal oscillator (VCXO)152. Furthermore, coupled to chip 10 is an external component 304 as well as body 306 which is configured to provide a plurality of signals which feed into a switch matrix such as switch matrix 20.

In all there is at least one preferred embodiment which includes a combined analog and digital circuit. The analog portion of the circuit is configured for applying an excitation current to the system with only 3 current levels at different frequencies. Next the analog portion is configured for detecting with a low-noise amplifier (LNA) the voltage drop from the system (input signal) caused by the excitation current. Next, the analog portion is configured to apply a frequency shift to the amplified input signal moving it to an intermediate frequency (f_{IF}), for example, by selecting f_{IF} > flicker noise corner frequency thus limiting the effects of noise. Next, the analog circuit is configured for low-pass filtering (LPF) and amplifying via a (PGA). At this point the frequency shifted signal is then converted to the digital domain via an (ADC).

Within the digital portion the signal is digitally demodulated and filtered such that the converted digital signal is used to obtain the I/Q DC values that represent the electrical impedance of the system.

Thus, in at least one embodiment, there is at least a first frequency down conversion in the analog domain and shifting the I/Q demodulation in the digital domain, which has the advantage of removing any sensible dual path from the analog domain with important benefits in terms of complexity, precision and power consumption.

In at least one embodiment, there is a phase-locked loop (PLL) module 161 that allows many of these processes to be synchronized in order to make multiple measurements and to extrapolate a matrix of values that give an accurate profile of the electrical impedance for the system under examination in a wider area.

Accordingly, while at least one embodiment of the present invention has been shown and described, it is to be understood that many changes and modifications may be made thereunto without departing from the spirit and scope of the invention as defined in the appended claims.

## Claims

1. PROCESS FOR MEASURING THE ELECTRICAL IMPEDANCE OF A SYSTEM,
**characterised in that**
it provides the following phases:
applying an excitation current to the system with only 3 current levels at different frequencies;
detecting with a low-noise amplifier (LNA) the voltage drop from the system (input signal) caused by the excitation current;
applying a frequency shift to the amplified input signal moving it to an intermediate frequency (f_{IF}), for example, by selecting f_{IF} > flicker noise corner frequency thus limiting the effects of noise;
low-pass filtering (LPF), amplifying (PGA), and then converting to the digital domain (ADC) the frequency shifted signal;
digitally demodulating and filtering the converted signal to obtain the I/Q DC values that represent the electrical impedance of the system.

2. PROCESS FOR MEASURING THE ELECTRICAL IMPEDANCE OF A SYSTEM, according to claim 1, **characterised in that** it comprises a first frequency down conversion in the analog domain and shifting the I/Q demodulation in the digital domain, has the advantage of removing any sensible dual path from the analog domain with important benefits in terms of complexity, precision and power consumption.

3. PROCESS FOR MEASURING THE ELECTRICAL IMPEDANCE OF A SYSTEM, according to claim 2, **characterised in that** it includes a phase-locked loop (PLL) module that allows many of these processes to be synchronized in order to make multiple measurements and to extrapolate a matrix of values that give an accurate profile of the electrical impedance for the system under examination in a wider area.

4. IMPEDANCE MEASURING CHIP, **characterised in that** it comprises:
at least one analog circuit comprising:
a plurality of analog inputs (10, 11, 12, 13);
at least one multiplexer (30) configured to received signals from said plurality of analog inputs (10, 11, 12, 13) wherein the analog circuit is configured to perform a first frequency down conversion;
at least one digital circuit comprising at least two demodulators (120, 122) configured to receive at least one signal from said at least one multiplexer (30) in said analog circuit, wherein said at least two demodulators are configured to shift an I, Q demodulation in said digital circuit.

5. IMPEDANCE MEASURING CHIP, according to claim 4, **characterised in that** it further comprises a switch matrix (20) configured to receive signals from said plurality of analog inputs, and at least one of a current sensor (22), a low noise amplifier (24), and a current control loop (23) coupled to an output of said switch matrix.

6. IMPEDANCE MEASURING CHIP, according to claim 4, **characterised in that** it further comprises a switch matrix (20), coupled to said plurality of analog inputs (10, 11, 12, 13) and at least one low noise amplifier (24) coupled to an output of said at least one switch matrix (20), and at least one current sensor (22) coupled to an output of said at least one switch matrix (20), and at least one multiplexer (30) coupled to an output of said current sensor (22) and said low noise amplifier (24).

7. IMPEDANCE MEASURING CHIP, according to claim 6, **characterised in that** it further comprises at least one first low pass filter (50) coupled to an output of said multiplexer (30).

8. IMPEDANCE MEASURING CHIP, according to claim 7, **characterised in that** it further comprises at least one programmable gain amplifier (52) coupled to an output of said low pass filter (50), wherein said at least one programmable gain amplifier (52) has an output that feeds into said at least two demodulators (64, 66).

9. IMPEDANCE MEASURING CHIP, according to claim 8, **characterised in that** it further comprises at least two second low pass filters (70, 72) wherein each of said demodulators (64, 66) has an output that feeds into at least one second low pass filter (70, 72).

10. IMPEDANCE MEASURING CHIP, according to claim 9, **characterised in that** at least one output of said synthesizer (60) is in the form of a voltage controlled current source (VCCS 40) that feeds into said switch matrix (40) and at least a second output of said synthesizer feeds into each of said at least two demodulators (64, 66).

11. IMPEDANCE MEASURING CHIP, according to claim 10, **characterised in that** it further comprises at least one additional multiplexer (74), wherein each of said second lower power filters (70, 72) feeds into said at least one additional multiplexer (74).

12. IMPEDANCE MEASURING CHIP, according to claim 11, **characterised in that** it further comprises at least one additional programmable gain amplifier (76), wherein said at least one additional multiplexer (74) has an output that feeds into said at least one additional programmable gain amplifier (76).

13. IMPEDANCE MEASURING CHIP, according to claim 4, **characterised in that** said analog circuit (31) further comprises at least one demodulator (100) and at least one digital to analog converter (130) which is configured to receive an input from said digital circuit (140), and wherein said digital to analog converter (130) has an output extending into said at least one demodulator (100) in said analog circuit (31).

14. IMPEDANCE MEASURING CHIP, according to claim 13, **characterised in that** said digital circuit comprises:
a first digital frequency domain circuit (140), comprising a clock synthesizer (154) and which operates at a highest frequency of any domain, and is responsible of generating all synchronous clocks and the digital sinusoid required by a dual step lock in operation;
a second digital frequency domain circuit (111), which comprises said at least two demodulators and which operates with maximal operating frequency of 10MHz;
a third frequency domain circuit (170), which contains all the components that work with a maximum frequency of 1MHz.
